# EUROPEAN PATENT SPECIFICATION

(11) **EP 2 606 827 B1**
(45) Date of publication and mention of the grant of the patent: **01.04.2015**
(21) Application number: 12196774.9
(22) Date of filing: 12.12.2012
(51) Int. Cl.: A61B 8/00, A61B 8/08, A61B 5/022, A61B 8/04

(54) **Cuff with ultrasonically transmissive portion and method of observing a tissue under pressure by using the same**
Druckmanschette mit ultraschalldurchlässigem Bereich und Verfahren zur Beobachtung eines mit Hilfe derselben mit Druck beaufschlagten Gewebes
Manchette avec une région perméable aux ultra-sons et procédé d'observation d'un tissu sous pression l'utilisant

(30) Priority: 21.12.2011 JP 2011279712; 31.10.2012 JP 2012240825
(43) Date of publication of application: 26.06.2013
(73) Proprietor: Nihon Kohden Corporation, Shinjuku-ku Tokyo (JP)
(72) Inventor: Ukawa, Teiji, Tokyo (JP); Takayanagi, Tsuneo, Tokyo (JP); Morimoto, Haruka, Tokyo (JP)
(74) Representative: Ilgart, Jean-Christophe

(56) References cited:
- EP-A2- 1 977 692
- WO-A1-01/00087
- WO-A2-2007/009118
- DE-A1-102005 012 655
- US-A- 3 356 086
- US-A1- 2005 054 958
- US-A1- 2008 262 347

## Description

### BACKGROUND

The presently disclosed subject matter relates to a cuff which is to be used in a blood pressure measurement or the like, and also to a method of observing a tissue under pressure by using it.

In the field of medicine, the central venous pressure (CVP) is sometimes measured during treatment of a critically ill patient. In such a measurement, a catheter is inserted into and indwelled in the superior or inferior vena cava of the patient, and the blood pressure in the region is measured. The measurement of the central venous pressure is performed in order to, for example, check whether the balance of the circulatory blood volume in the living body of the patient is lost because of bleeding, dehydration, or the like, or whether heart failure or the like occurs or not.

As described above, the related-art measurement of the central venous pressure is performed while a catheter is inserted into and indwelled in the body of the patient. Therefore, the physical burden on the patient is large. Consequently, it is requested to develop a less or non-invasive method of measuring the central venous pressure.

On the other hand, Non-patent Document 1 below discloses a technique in which an ultrasonic probe in which a pressure sensor is attached to the tip end is pressed against a region of the forearm, the state of the vein in the region is checked on a monitor, the pressure at the timing when the vein collapses is measured, and the pressure is determined as a pressure (hereinafter, referred to as the alternative central venous pressure) that functions as an alternative to the central venous pressure.

Non- or less-invasive medical techniques have been developed also in fields other than the measurement of the central venous pressure. Examples of such techniques are the apparatus for examining a breast disclosed in Patent Document 1, and the method which is described in Non-patent Document 2 and which is to be used in the diagnosis of an ectopic ureterocele.

The apparatus for examining a breast disclosed in Patent Document 1 is used in breast cancer screening or the like. The apparatus for examining a breast has: an ultrasonic array probe; a sealing liquid container which accommodates liquid and the ultrasonic array probe, and which partly has a stretchable film through which an ultrasonic wave is transmissible; a contact pressure adjusting unit which, in accordance with the a specimen, adjusts a contact pressure between the specimen and the stretchable film; an ultrasonic wave transmitting/receiving circuit which causes the ultrasonic array probe to transmit and receive an ultrasonic wave; an image producing unit which produces an image of the specimen from a signal that is received by the ultrasonic wave transmitting/receiving circuit; and an image displaying unit which displays the produced image. According to the configuration, breasts of various sizes can be pressed into a shape which is adequate for displaying an image.

The method of Non-patent Document 2 is used in diagnosis of disease which is called an ectopic ureterocele, and in which an ureterocele develops in a state where the ureter dose not open in the trigone of the bladder. According to the method of Non-patent Document 2, in the case where an ultrasonic examination is performed in a state where the hydroureter in the flank is pressed, in order to diagnose an ectopic ureterocele, when an ectopic ureterocele exists, the ureterocele can be clearly displayed in an echo image due to an ultrasonic wave.

### (Patent Document 1) JP-A-2007-282960

(Non-patent Document 1) Christoph Thalhammer et al. "Noninvasive Central Venous Pressure Measurement by Controlled Compression Sonography at the Forearm," Journal of the American College of Cardiology, Elseveir Inc., [online], January 17, 2011, Vol. 50, No. 16, p. 1584-1589, Internet <URL:http://content.onlinejacc.org/cgi/content/full/50/16/1584>

(Non-patent Document 2) Gotaro KURASAWA et al. "Sokufukubu Appakuho niyoru Choonpa Kensa ga Shindan ni Yuyodeatta Isyosei Nyokanryu no Ichirai," Nippon Syoni Jinzobyo Gakkai Zashi, Nippon Syoni Jinzobyo Gakkai, 1996, Vol. 9, No. 1, p. 49-52

In the method of Non-patent Document 1, a pressure sensor-equipped ultrasonic probe pressurizes the vein in the forearm in the range of from 0 mmHg to 50 mmHg at intervals of 5 mmHg, and the pressure at the timing when the blood vessel collapses is determined as a pressure corresponding to the central venous pressure. In the method of Non-patent Document 1, however, the probe is pressed by the hand, and therefore the pressure is hardly maintained constant. Consequently, it is difficult to accurately measure the alternative central venous pressure.

A cuff is used for measuring the blood pressure of the artery. If a cuff is used, the whole periphery of the vein can be constantly pressurized also in measurement of the alternative central venous pressure (the pressure is not limited to the venous pressure in the forearm, and means the venous pressure of the vein in a region where the pressure can be measured by using a cuff, and the same shall apply hereinafter). However, a related-art cuff cannot observe a tissue such as the vein under pressure, and therefore cannot measure the alternative central venous pressure.

In the examining apparatus disclosed in Patent Document 1, the structure of the sealing liquid container is complicated, and therefore a dedicated apparatus is necessary. If such a dedicated apparatus is necessary in the examination of breast cancer, an economic burden is imposed on a hospital which introduces the apparatus, and a patient who uses the apparatus. Therefore, the breast cancer screening cannot be easily performed.

In Non-patent Document 2, the manner of pressing the hydroureter in the flank is not described in detail. When an apparatus for pressing the flank is not prepared, a technician such as a doctor presses the region. In the case where, in order to display an ureterocele, a technician manually presses the hydroureter in the flank with the hand, however, it is difficult to apply a constant pressing force, and therefore a clear display of the hydroureter can be hardly obtained.

Documents US 2008/262347 A1 and WO 01/00087 A1 disclose a cuff according to the preamble of claim 1.

### SUMMARY

The presently disclosed subject matter may provide a cuff which enables the state of a tissue under pressure to be observed, and an examination or the like to be easily performed, and a method of observing a tissue under pressure by using it.

The cuff may comprise: a belt-like body which is adapted to be wrapped around a desired region of a living body; a fixing portion which is provided in the body, and which is configured to fix the body in a state where the body is adapted to be wrapped around the region of the living body; a pressurizing portion which is provided in the body, and to which a fluid pressure is to be supplied to be inflated to pressurize the region of the living body; and an ultrasonic transmissive portion which is provided in the body, and through which an ultrasonic wave for observing a tissue in the region of the living body is transmissible. The ultrasonic transmissive portion is configured by providing, in the body, a window portion formed by a member having ultrasonic transmissive properties.

The region of the living body may be one of four limbs.

The region of the living body may be breast, and the fixing portion may be configured to fix the body in a state where the body is adapted to be wrapped around a chest so as to cover the breast.

The region of the living body may be abdomen.

In an embodiment not forming part of the invention, the ultrasonic transmissive portion may be configured by forming the body by a member having ultrasonic transmissive properties.

The ultrasonic transmissive portion may be disposed in a position through which an extension line of a centerline of the pressurizing portion in a direction intersecting with a direction of wrapping the body around the region of the living body passes.

The ultrasonic transmissive portion may be visually transparent.

The tissue observing method may comprise: wrapping the body of the cuff around the region of the living body; fixing, by the fixing portion, the body in a state where the ultrasonic transmissive portion faces against an observation region of the region of the living body; supplying the fluid pressure to the pressurizing portion to pressurize the region of the living body; and irradiating an ultrasonic wave from an ultrasonic probe through the ultrasonic transmissive portion to observe a tissue of the observation region of the region of the living body.

### BRIEF DESCRIPTION OF THE DRAWINGS

Fig. 1 is a block diagram showing a control system of a blood pressure measuring apparatus which uses a cuff of Embodiment 1 of the presently disclosed subject matter.
Fig. 2 is a perspective view showing the cuff.
Fig. 3A is a view showing a rear surface which is on the inner side when the cuff is wrapped around a measurement region.
Fig. 3B is a sectional view taken along line 3B-3B in Fig. 3A.
Fig. 4 is a view showing a front surface which is on the outer side when the cuff is wrapped around the measurement region.
Fig. 5 is a view showing an air bag.
Fig. 6 is a sectional view illustrating an experiment in which pressures were measured that act respectively on a point a which is between an arm region and the air bag of the cuff of Embodiment 1 in which a window portion formed by an ultrasonic transmissive film is placed in a body, and on a point b which is between the ultrasonic transmissive film and the arm region.
Fig. 7A is a view showing a result of an experiment in which a blood pressure was measured by the cuff of Embodiment 1 in which the window portion formed by the ultrasonic transmissive film is placed in the body.
Fig. 7B is a view showing a result of an experiment in which a blood pressure was measured by the cuff of Embodiment 1 in which the body is formed by the ultrasonic transmissive film.
Fig. 8 is a front view of a human body.
Fig. 9 is a sectional view taken along line 9-9 in Fig. 8 showing a state where a cuff of Embodiment 2 of the presently disclosed subject matter is wrapped.
Fig. 10 is a sectional view taken along line 10-10 in Fig. 8 showing a state where a cuff of Embodiment 3 of the presently disclosed subject matter is wrapped.

### DETAILED DESCRIPTION OF EXEMPLARY EMBODIMENTS

Hereinafter, embodiments of the presently disclosed subject matter will be described with reference to the accompanying drawings. The following description shall not be interpreted to limit the technical scope and meaning of terms of the claims. In the drawings, the dimension ratios are exaggerated for the sake of convenience in description, and may be sometimes different from the actual ratios.

### (Embodiment 1)

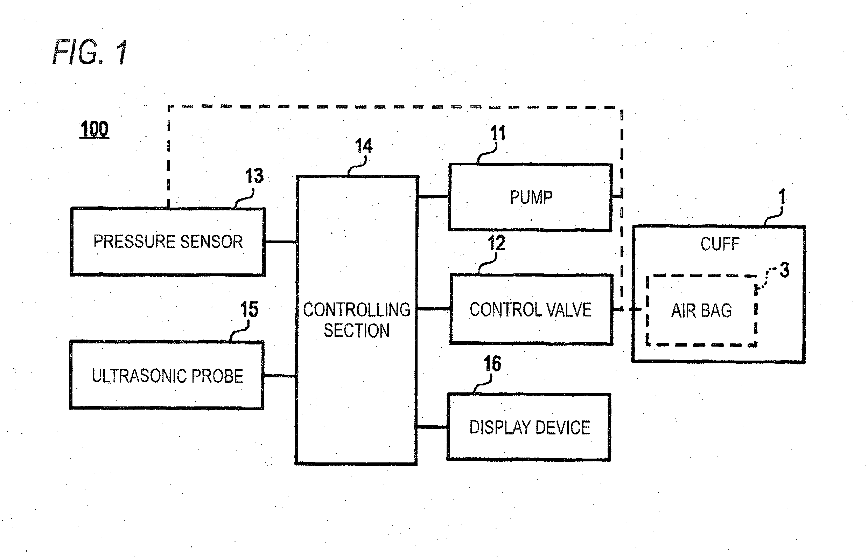

Fig. 1 is a block diagram showing a control system of a blood pressure measuring apparatus 100 which uses a cuff of Embodiment 1 of the presently disclosed subject matter. The blood pressure measuring apparatus 100 in Embodiment 1 is a blood pressure measuring apparatus of the oscillometric type, and applies a calculation process on a pressure wave pulse acquired during depressurization of the cuff to calculate the maximal and minimal blood pressures and the like. The blood pressure measuring apparatus 100 includes the cuff 1, a pump 11, a control valve 12, a pressure sensor 13, a controlling section 14, an ultrasonic probe 15, and a display device 16.

The pump 11 supplies a predetermined flow amount of the air to an air bag 3 which is housed in the cuff 1, and which is described later. The control valve 12 is controlled by the controlling section 14 to switch air supply/exhaustion to and from the air bag 3. The pressure sensor 13 detects the pressure applied to the air bag 3, and sends a signal indicative of the pressure to the controlling section 14. The controlling section 14 calculates the pressure applied to a desired region of the living body such as the upper or lower limb from the pressure signal obtained from the pressure sensor 13, and controls the flow amount to be supplied from the pump 11.

When the process of measuring the pressure is ended, the controlling section 14 controls the control valve 12 to exhaust the air in the air bag 3, and then causes the pump 11 to stop. Independent from the pressure measurement, the controlling section 14 performs an image process on a signal which is obtained by touching the ultrasonic probe 15 against a measurement region, and controls the display device 16 to display an echo image. The display device 16 displays various results such as the maximal and minimal blood pressures, the echo image, and the like which are processed by the controlling section 14. Alternatively, the display device 16 may be configured by a device on which the blood pressure is displayed, and another device on which the echo image is displayed.

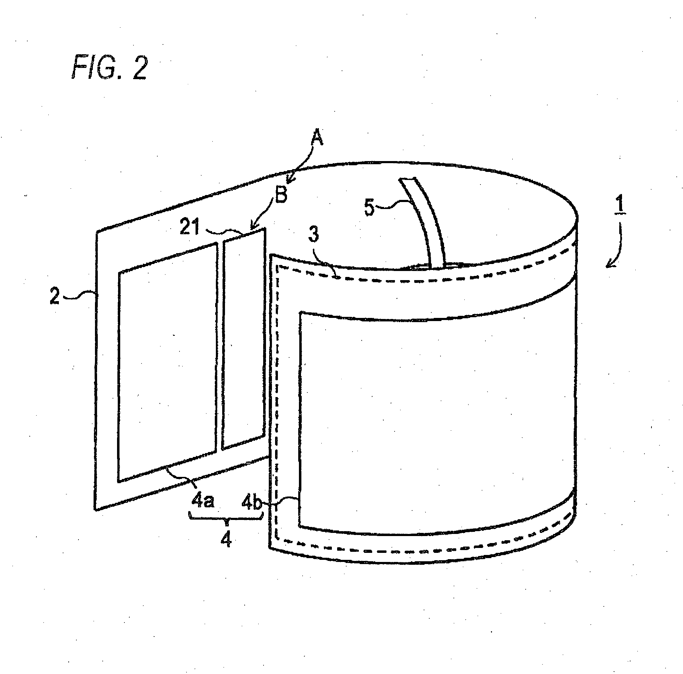

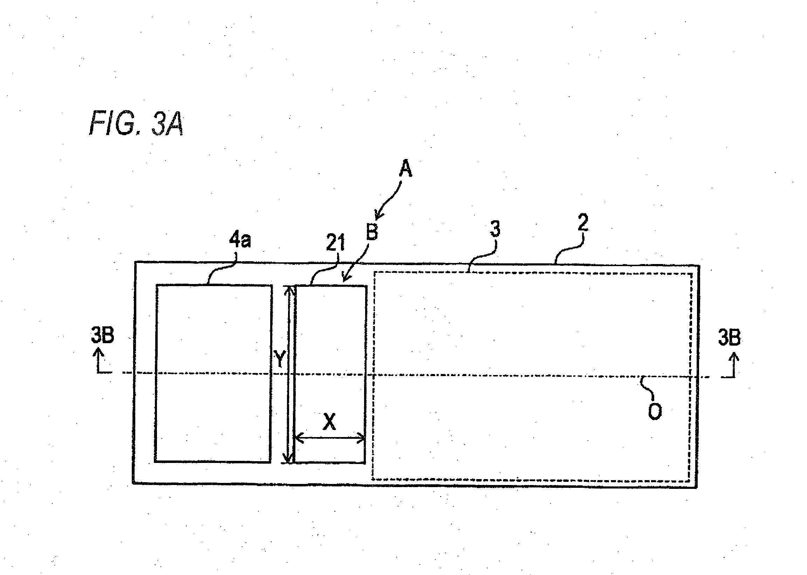

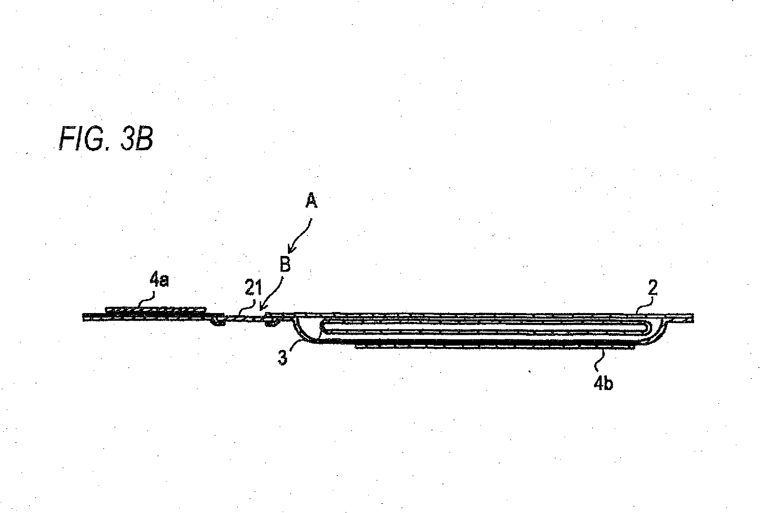

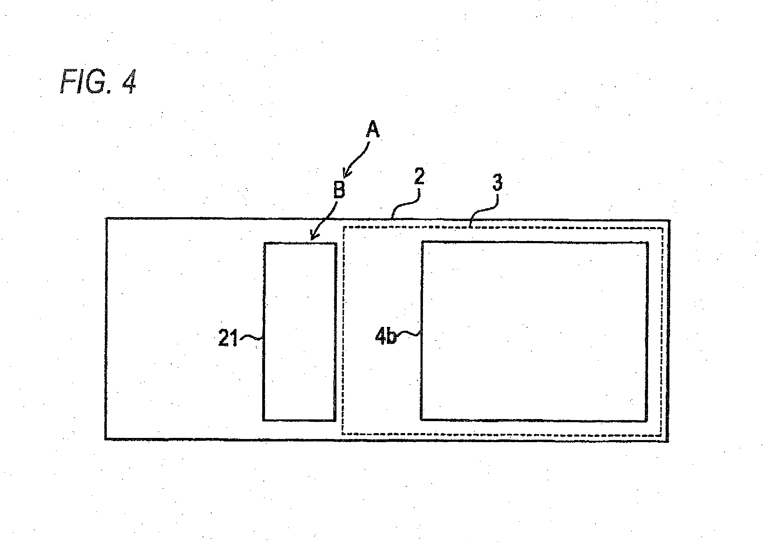

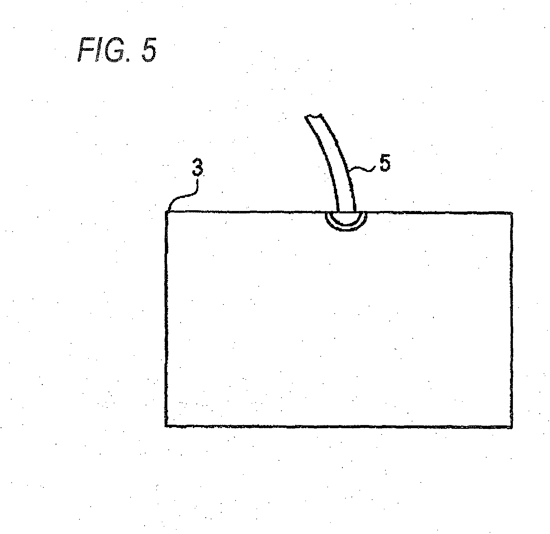

Fig. 2 is a perspective view showing the cuff of Embodiment 1 of the presently disclosed subject matter, Fig. 3A is a view showing the rear surface which is on the inner side when the body of the cuff is wrapped, Fig. 3B is a sectional view taken along line 3B-3B in Fig. 3A, and Fig. 4 is a view showing the front surface which is on the outer side when the cuff is wrapped around the measurement region. Fig. 5 is a view showing the air bag.

Referring to Fig. 2, generally speaking, the cuff 1 has: a belt-like body 2 which is to be wrapped around a desired region of the living body; a fixing portion 4 which is disposed in the body 2, and which fixes the body 2 to a state where it is wrapped around the desired region of the living body; the air bag 3 (corresponding to a pressurizing portion) which is disposed in the body 2, and which is inflated by supplying the air to pressurize the desired region of the living body; and an ultrasonic transmissive portion A which is disposed in the body 2, and through which an ultrasonic wave for observing a tissue in the desired region of the living body is transmissible. Hereinafter, they will be described in detail.

As shown in Figs. 3A and 4, the body 2 is configured so as to have a belt-like shape (rectangular shape) when the body is developed. The body 2 is formed by, for example, fibers of a synthetic resin such as polyester or nylon. A housing pocket for housing the air bag 3 is formed in the body 2. As shown in Fig. 3B, in a part of the body 2, an ultrasonic transmissive film 21 constituting the ultrasonic transmissive portion A is placed while forming a window portion B.

The fixing portion 4 is configured by a surface fastener. The surface fastener includes a hook surface 4a which is disposed in a left end portion in Fig. 3A, and a loop surface 4b which is disposed in a right end portion in Fig. 4.

The hook surface 4a and the loop surface 4b are formed by cutting away respective necessary amounts from roll-like members, and then sewn to the body 2. In the embodiment, the hook surface 4a is placed on the side which is inside when the cuff 1 is wrapped, and the loop surface 4b is placed on the outer side. Alternatively, the hook surface 4a may be placed on the outer side, and the loop surface 4b may be placed on the inner side.

As shown in Fig. 3B, the air bag 3 is housed in the housing pocket of the body 2 so that, when inflated, the bag presses the blood pressure measurement region of a blood vessel. Since the air bag 3 is internally housed in the body 2, the bag is indicated by broken lines in Figs. 2, 3A, and 4.

The air bag 3 is placed in a right end portion in Fig. 3A so that, when the body 2 is wrapped around the pressure measurement region, the body is prevented from being placed on the inner side with respect to the air bag 3 to impede the pressurization. The cuff 1 is attached to the patient by being wrapped around the measurement region with starting from the right end portion in Fig. 3A where the air bag 3 is placed. The length of the air bag 3 in the direction of wrapping of the cuff 1 is set so that the air bag and the ultrasonic transmissive portion A cooperate to approximately cover the whole periphery of the measurement region.

As shown in Fig. 5, a tube 5 which is connected to the control valve 12 is joined to one side of the air bag 3. The tube 5 is joined to the air bag 3 in such a manner that, when the air bag 3 is housed in the body 2, the tube is located in the portion of the housing pocket of the body 2.

In the illustrated embodiment, the ultrasonic transmissive portion A is configured by placing the window portion B formed by a member having ultrasonic transmissive properties, in the body 2. As the member having ultrasonic transmissive properties, the ultrasonic transmissive film 21 is used. The material forming the ultrasonic transmissive film 21 is not particularly limited. For example, polymethylpentene which is called TPX (registered trademark) may be used.

As shown in Fig. 3A, the window portion B formed by the ultrasonic transmissive film 21 is placed between the air bag 3 and the hook surface 4a in the direction of wrapping of the cuff 1 so as not to overlap with the air bag 3, the hook surface 4, and the loop surface 4b. When the air is interposed, an ultrasonic wave is reflected thereby, and an echo image cannot be produced. When the ultrasonic transmissive film 21 is disposed between the air bag 3 and the hook surface 4a, therefore, an echo image can be surely produced.

As shown in Fig. 3A, preferably, the ultrasonic transmissive portion A is placed in a position where an extension line of the centerline O, which is parallel to the direction of wrapping of the body 2, of the air bag 3 in a direction intersecting with the direction of wrapping of the body 2 passes, because of the following reason. In the case where pressurization is performed by the air bag 3, even when the air bag 3 is configured by a material which is easily inflatable, an end portion of the air bag 3 cannot inflate in the same degree as a middle portion. When the ultrasonic transmissive film 21 is placed so as to pass through the extension line of the centerline O of the air bag 3, the ultrasonic transmissive film 21 is placed in a portion where a blood vessel can be sufficiently pressurized, so that the state of the blood vessel can be adequately checked.

Moreover, it is preferable that the ultrasonic transmissive portion A is transparent, because of the following reason. According to the configuration, the measurement region can be viewed from the above the ultrasonic transmissive film 21, and the convenience in the case where the cuff 1 is to be adjusted to the position of the measurement region can be improved.

Referring to Fig. 3A, the length X of the window portion B in the direction of wrapping of the cuff 1 is not particularly limited. However, for example, the length may be determined in consideration of the size of a tip end portion of the ultrasonic probe 15. Also the length Y of the window portion B in the width direction intersecting with the direction of wrapping of the cuff 1 is not particularly limited. However, for example, the length may be determined in consideration of the distance along which the probe 15 is to be moved. In one example, the length X of the window portion B is about 20 mm, and the length Y is about 50 mm. The ultrasonic transmissive film 21 has a thickness of, for example, about 0.1 mm.

The blood pressure measurement using the cuff 1 of the embodiment will be described taking as an example the case where the venous pressure in the right forearm is measured as the alternative central venous pressure. First, the cuff 1 is wrapped around the right forearm of the patient to be attached thereto, and the cuff 1 is adjusted by rotating it so that the portion where the ultrasonic transmissive film 21 is formed is above the position of the vein which is the measurement object.

After the portion of the cuff 1 where the ultrasonic transmissive film 21 is formed is located above the vein, a gel through which an ultrasonic wave is transmissible is applied onto the ultrasonic transmissive film 21. The gel may be further applied between the ultrasonic transmissive film 21 and the forearm region which is the measurement region.

While touching the ultrasonic probe 15 against the ultrasonic transmissive film 21 to which the gel is applied, the air bag 3 of the cuff 1 is inflated by the pump 11 to pressurize the measurement region, and the condition of the blood vessel is observed on the display device 16.

In the related-art measurement of the alternative central venous pressure, a pressure sensor is attached to a probe, and a pressure is applied by the hand. Therefore, it is difficult to pressurize the vein at a constant pressure. Even by using a related-art cuff, a tissue under pressure cannot be observed, and the alternative central venous pressure cannot be measured.

In the cuff 1 of the embodiment, by contrast, the ultrasonic transmissive film 21 is disposed in the cuff 1, and therefore, while the whole periphery of the measurement region is evenly pressurized at a constant pressure, the state of a tissue under pressure can be observed. Consequently, it is possible to measure the alternative central venous pressure.

The cuff 1 of the embodiment can be used also in an index which is called ABI, and in which a blood pressure measurement is employed. The ABI (Ankle Brachial Index) indicates the ratio of the maximal blood pressure of the ankle to that of the upper arm, and is also called the ratio of ankle pressure to brachial pressure.

The ABI is obtained by measuring the blood pressures of the upper arm and the ankle in a supine position. In a healthy person, the blood pressure of the ankle is slightly higher than that of the upper arm. In the case where a constricted or obstructed part exists in a blood vessel of the ankle, the blood flow is impaired, and the blood pressure of the ankle is lowered as compared to that of the upper arm, so that the ABI shows a low value. The ABI is measured in the first inspection of arteriosclerosis obliterans.

Even in the case where a constricted part exists in a blood vessel of the foot, when calcification occurs in a vessel of the blood pressure measurement region of the ankle, the vessel is hardly crushed even by pressurization of a cuff, and therefore the situation where the ABI does not show a low value is produced. In the case where calcification occurs as described above, when a blood pressure measurement is performed by using a related-art cuff, it is impossible to adequately determine whether arteriosclerosis obliterans occurs or not.

Even in such a case, according to the cuff 1 of the embodiment, an ultrasonic echo image of a vessel region of the foot can be produced, so that the state of the tissue of the measurement region under pressure can be observed. Therefore, it is possible to check whether calcification occurs in the measurement region or not, and the reliability of the ABI can be improved.

As described above, the cuff 1 of the embodiment is configured so as to have: the body 2; the fixing portion 4 which is disposed in the body 2, and which fixes the body 2 to a state where it is fixed to the desired region of the living body such as the forearm; the air bag 3 which is disposed in the body 2, and which is inflated by supplying a fluid pressure to pressurize the desired region of the living body; and the ultrasonic transmissive portion A which is disposed in the body 2, and through which an ultrasonic wave for observing a tissue in the desired region of the living body is transmissible. Therefore, the ultrasonic transmissive portion A enables the state of a tissue under pressure to be observed.

The cuff 1 is wrapped around the desired region of the living body, the ultrasonic transmissive portion A is caused to face against an observation region, the body 2 is fixed to the desired region of the living body by the fixing portion 4, and the desired region of the living body is pressurized by the air bag 3. Then, the ultrasonic wave irradiated from the ultrasonic probe 15 is caused to transmit through the ultrasonic transmissive portion A to impinge on the observation region, thereby enabling a measurement of a blood pressure such as the alternative central venous pressure to be accurately performed.

The ultrasonic transmissive portion A in the embodiment is configured by placing the window portion B formed by the ultrasonic transmissive film 21. When a window portion is disposed in a related-art cuff and an ultrasonic transmissive film is placed in the portion, therefore, it is possible to form a cuff which enables the state of a tissue under pressure to be observed.

The ultrasonic transmissive portion A is placed so that an extension line of the centerline O, which is parallel to the direction of wrapping the body 2 around the desired region of the living body, of the air bag 3 in the direction intersecting with the direction of wrapping the body 2 around the desired region of the living body passes through the ultrasonic transmissive portion A. In the cuff 1, therefore, the ultrasonic transmissive portion A can be placed in a location where a blood vessel can be sufficiently pressurized, so that the state of the blood vessel can be adequately checked.

Since the ultrasonic transmissive portion A is transparent, the ultrasonic transmissive portion A can be adjusted to the measurement region by means of a visual check. Therefore, the convenience in the case where the cuff 1 is to be attached to the measurement region can be improved.

The invention is not limited to the above-described embodiment, and can be variously modified within the scope of the appended claims.

According to the invention the ultrasonic transmissive portion A is configured by placing the window portion B formed by the member having ultrasonic transmissive properties in the body 2 as has been described in the embodiment . In an alternative not forming part of the invention, the ultrasonic transmissive portion may be configured by forming the body by a member having ultrasonic transmissive properties. According to the latter configuration, the range where the ultrasonic probe 15 can be placed can be expanded as compared to the case where a member having ultrasonic transmissive properties is partly used, and therefore the position adjustment of the cuff can be facilitated.

The embodiment in which the housing pocket for housing the air bag 3 is formed in the body 2 has been described. The housing pocket is required simply to house the air bag 3. In the case where the pocket is formed by overlapping two sheets of the material constituting the body 2, such as polyester, therefore, the other portion including the hook surface 4a and the like may be configured by one sheet of the material. This is applicable also to a cuff in which the body is formed by an ultrasonic transmissive film.

Although the embodiment in which the ultrasonic transmissive film 21 is configured by polymethylpentene which is called TPX or the like has been described, the invention is not limited to this, and another configuration may be employed. In order to prevent an ultrasonic wave from being reflected from an intermediate point, the ultrasonic transmissive film 21 may be configured by a material which is similar in acoustic impedance to the body tissue.

The embodiment in which the ultrasonic wave transmission portion A is configured so as to be transparent has been described. Since, among materials through which an ultrasonic wave is transmissible, there are those which are not transparent, the ultrasonic wave transmission portion A may not always be transparent.

Next, an experiment in which the cuff of the embodiment was used, and which was conducted with respect to a pressure acting on a region immediately below the ultrasonic transmissive film will be described.

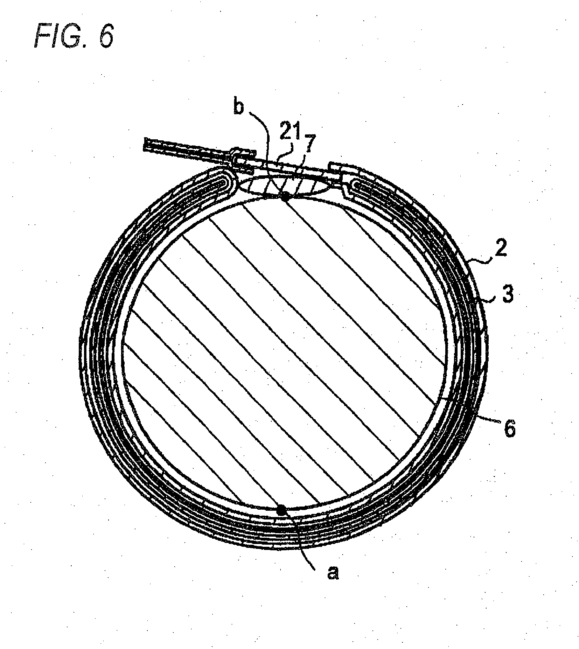

Fig. 6 is a sectional view illustrating the experiment in which pressures were measured that act respectively on a point a which is between an arm region that is the upper arm, and the air bag of the cuff in which the window portion formed by the ultrasonic transmissive film is placed in the body, and on a point b which is between the ultrasonic transmissive film and the arm region.

In the experiment, a cuff in which a window portion formed by an ultrasonic transmissive film is placed in the body, and that in which the body is formed by an ultrasonic transmissive film were used. As shown in Fig. 6, the cuff 1 is wrapped around the arm region 6, and a pressure acting on the point a between the air bag 3 and the arm region 6, and that acting on the point b between the ultrasonic transmissive film and the arm region 6 were compared with each other.

The pressure acting between the air bag 3 and the arm region 6 was detected by the pressure sensor 13 shown in Fig. 1, and output as a pressure waveform.

By contrast, with respect to the pressure immediately below the ultrasonic transmissive film, when the pressure is detected while placing a bag filled with air between the ultrasonic transmissive film and the arm region 6, the bag filled with air collapses at a pressure of a certain level or higher, and it is impossible to detect a pressure which is higher than the level. Therefore, a bag 7 having a size which is similar to that of the ultrasonic transmissive film was filled with water, and placed between the ultrasonic transmissive film and the arm region 6. A pressure sensor was attached to the bag 7 filled with water, and a pressure waveform was output.

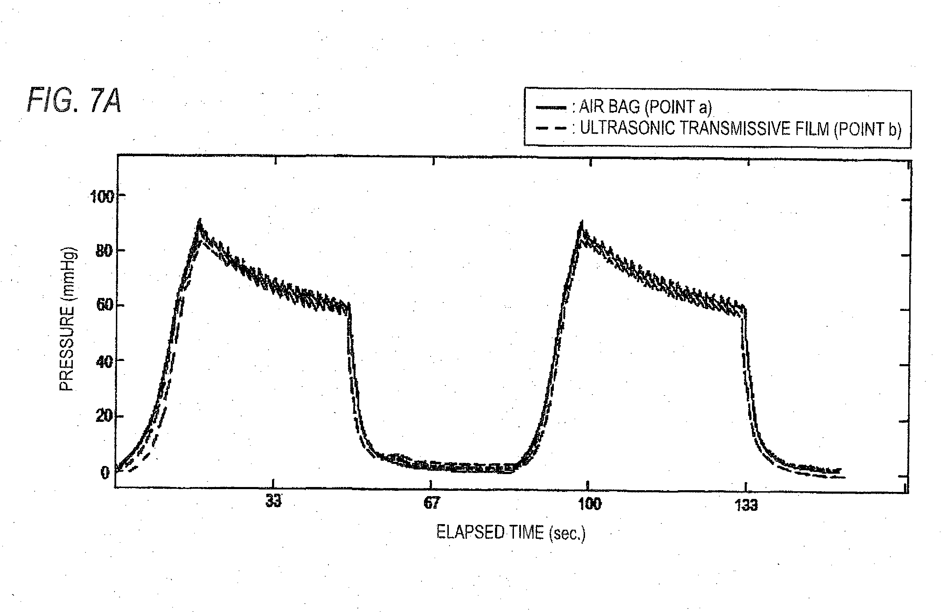

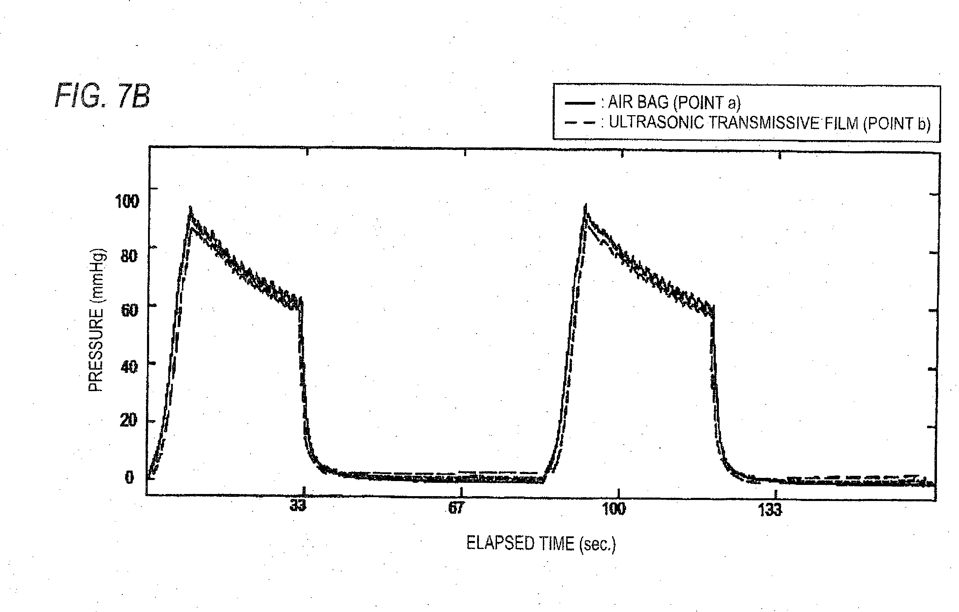

Fig. 7A is a chart of waveforms output in the case where the blood pressure was measured by the cuff in which the window portion formed by an ultrasonic transmissive film is placed in the body, and Fig. 7B is a chart of waveforms output in the case where the blood pressure was measured by the cuff in which the body is formed by an ultrasonic transmissive film. The solid-line parts indicate the waveform of the pressure at the point a between the air bag and the arm region, and the broken-line parts indicate the waveform of the pressure at the point b between the ultrasonic transmissive film and the arm region.

The solid-line part and the broken-line part in each of the figures substantially overlap with each other, and therefore the broken-line part is additionally drawn in the vicinity of the solid-line part. The rates of pressurizing and depressurizing the arm region in Fig. 7A are different from those in Fig. 7B. However, the pressure values to be applied are equal to each other, and the pressurizing and depressurizing operations are performed two times. Even when the rates are different from each other, therefore, the experimental results are not affected. As shown in Figs. 7A and 7B, it was confirmed that, during depressurization, an amplitude waveform due to the blood flow appears in both the cases.

As shown in Fig. 7A, it was confirmed that, even when the pressure acting between the air bag 3 and the arm region 6 is compared with that between the ultrasonic transmissive film and the arm region 6, there is no substantial difference between them, and they are identical to each other. This is applicable also in the case of Fig. 7B. Consequently, it was confirmed that, by using a cuff in which a window portion formed by an ultrasonic transmissive film is placed in the body, or that in which the body is formed by an ultrasonic transmissive film, the state of a tissue under pressure can be observed while maintaining the accuracy of the blood pressure measurement.

### (Embodiment 2)

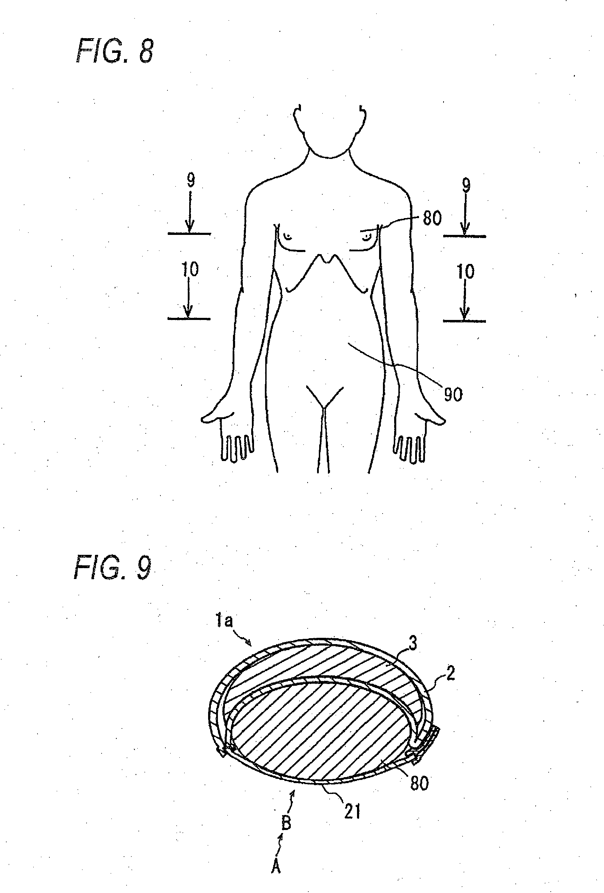

Next, Embodiment 2 of the presently disclosed subject matter will be described. Fig. 8 is a front view of a human body, and Fig. 9 is a sectional view taken along line 9-9 in Fig. 8 showing a state where a cuff of Embodiment 2 of the presently disclosed subject matter is wrapped.

In Embodiment 1 above-described, when the cuff 1 is used in a limb region such as the upper limb or the lower limb, the alternative central venous pressure can be accurately measured while pressurizing the region at a constant pressure, and, when the cuff is used in the blood pressure measurement for calculating the ABI, the reliability of the ABI can be improved. Alternatively, the cuff of the presently disclosed subject matter can be used in the following manner. The components identical with those of Embodiment 1 are denoted by the same reference numerals, and their detailed description will be omitted.

A cuff 1a of Embodiment 2 is used in breast cancer screening. The cuff 1a of Embodiment 2 is wrapped around the chest 80, and the breasts are pressed at a predetermined pressure by the inflation of the air bag 3.

The circumferential length of the body 2 is adjusted so as to be suitable for the size of the chest of an adult female. The circumferential length of the ultrasonic transmissive portion A is adjusted to a degree at which the portion can cover the breasts when the cuff 1a is attached. Although one ultrasonic transmissive portion A is disposed as shown in Fig. 9, two ultrasonic transmissive portions may be disposed in accordance with the breasts. In the case where the body 2 is formed by an ultrasonic transmissive film, the window portion B may not be disposed.

The length of the body 2 in the direction perpendicular to the sheet of Fig. 9 is adjusted to a degree at which the body can cover the whole breast also in the direction. The radial thickness in the case where the air bag 3 is filled with air or the like is adjusted so as to be suitable for the size of the breast of an individual person. The circumferential length of the air bag 3 is adjusted so as not to overlap with the ultrasonic transmissive portion A when the cuff 1a is attached to the patient. The air bag 3 is adjusted so as to, when attached to the patient, be placed on the back side and/or lateral side of the chest excluding the breasts (see Fig. 9).

Breast cancer screening using the cuff 1a of Embodiment 2 is performed in the following manner. The cuff 1a is wrapped around the chest 80 of the patient so that the ultrasonic transmissive portion A covers both the breasts, and then fixed in a state where a predetermined circumferential length is set. Next, the air bag 3 is inflated by the pump 11. Since the circumferential length of the cuff 1a is fixed by the fixing portion 4, the chest 80 is pressed, and also the breasts are similarly pressed, with the result that the breasts are deformed to a substantially flat state. In this state, the ultrasonic probe 15 is touched against the breast, and the internal condition of the breast is observed in the echo image displayed on the display device 16, thereby determining whether tumor exist or not.

In breast cancer screening, in order to find tumor, the ultrasonic probe is touched against all regions of the breast, and the internal condition is observed. In the breast, the central and peripheral regions have different thicknesses. When, in the breast, a region having a relatively large thickness is not properly collapsed, therefore, there is a case where an echo image of the region cannot clearly display deep tissue.

By contrast, according to the cuff 1a of Embodiment 2, also an echo image of a breast region having a relatively large thickness is enabled to be clearly displayed so that the condition of internal tissue can be observed, simply by the improvement that, in the cuff having a size which allows the cuff to be wrapped around the chest, the ultrasonic transmissive portion is disposed in accordance with the size of the breasts, and the air bag is adjusted so as not to overlap with the ultrasonic transmissive portion. As the ultrasonic probe which is to be touched against the ultrasonic transmissive portion A, and the like, moreover, related-art devices can be used. When the cuff 1a of Embodiment 2 is used, therefore, breast cancer screening can be easily performed.

As described above, the cuff 1a of Embodiment 2 is configured so that it can be fixed by the fixing portion 4 in a state where the body 2 is wrapped around the chest 80 so as to cover the breasts. When the cuff 1a of Embodiment 2 is used, therefore, breast cancer screening can be easily performed by using a related-art ultrasonic apparatus without necessitating a dedicated apparatus.

### (Embodiment 3)

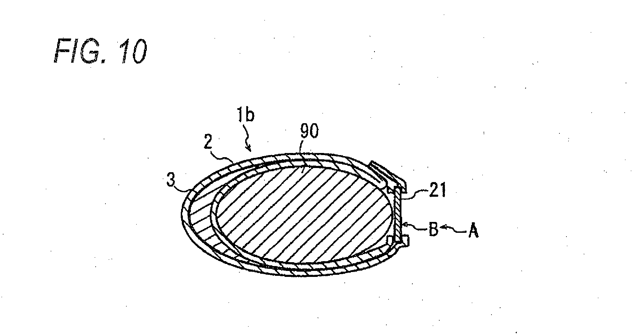

Next, Embodiment 3 of the presently disclosed subject matter will be described. Fig. 10 is a sectional view taken along line 10-10 in Fig. 8 showing a state where a cuff of Embodiment 3 is attached to the abdomen.

The cuff of Embodiment 1 is attached to a limb region to be used in the blood pressure measurement for measuring the central venous pressure or for calculating the ABI, and the cuff of Embodiment 2 is attached to the chest to be used in breast cancer screening. In addition to above-described uses, the cuff of the presently disclosed subject matter can be used also in the following manner.

The cuff 1b of Embodiment 3 is used in, for example, the diagnosis of an ectopic ureterocele. In the cuff 1b of Embodiment 3, the circumferential length of the body 2 is adjusted so that the body can be wrapped around the abdomen 90. The circumferential length of the ultrasonic transmissive portion A is adjusted to a degree at which a wide region of the abdomen 90 can be observed, and the pressing by the air bag 3 can be sufficiently performed.

Similarly with the above-described embodiments, the circumferential length of the air bag 3 is adjusted to a degree at which the bag does not overlap with the ultrasonic transmissive portion A. The air bag 3 is placed in a region where the pressing is performed when the cuff 1b is attached to the patient as shown in Fig. 10, or, in the embodiment, in a position which is to be butted against the flank.

For example, the lengths of the body 2 and the air bag 3 in the direction perpendicular to the sheet of Fig. 10 are adjusted to respective degrees at which they can cover the flank in the direction. However, the lengths are not limited to the above. Their radial thicknesses in the case where the air bag 3 is filled with air or the like are adjusted in accordance with the waist circumference of the patient.

As described above, cases were reported in which, in an ultrasonic examination which is performed in the diagnosis of an ectopic ureterocele, when the ultrasonic examination is performed in a state where the hydroureter in the flank is pressed, the ureterocele can be clearly displayed.

Also in the above cases, when the cuff 1b of Embodiment 3 is used, the ectopic ureterocele can be clearly displayed. Namely, the body 2 of the cuff 1b is wrapped around the patient so that the air bag 3 is butted against the hydroureter in the flank, the body is fixed by the fixing portion 4, and the air bag 3 is pressurized to a predetermined pressure by the pump 11 to be inflated. In this state, the ultrasonic probe 15 is butted against the ultrasonic transmissive portion A, and the condition of the bladder is observed on the display device 1.

In the pressurization by the air bag 3, a constant pressing force can be stably applied to the flank as compared to the case where the pressurization is performed by the hand. The pressure application on the air bag 3 by the pump 11 can be adjusted while viewing an indicator to check the current pressure, and therefore the pressing force can be accurately adjusted. In the pressing by the cuff, a dedicated apparatus is not used. Therefore, the diagnosis of an ectopic ureterocele or the like can be easily performed simply by the improvement that the ultrasonic transmissive portion is disposed in a related-art cuff, and the air bag is formed so as not overlap with the ultrasonic transmissive portion.

In the cuff 1b of Embodiment 3, as described above, the size is adjusted so that the body 2 is fixed by the fixing portion in the state where the body is wrapped around the abdomen 90 so that the air bag 3 is butted against the hydroureter in the flank. Therefore, the pressing force can be constantly applied as compared to the manual case, and accurately controlled, thereby enabling an ureterocele to be smoothly displayed in the diagnosis of an ectopic ureterocele or the like. Also in the cuff 1b of Embodiment 3, the diagnosis of an ectopic ureterocele or the like can be easily performed without using a dedicated apparatus, simply by the improvement that the ultrasonic transmissive portion is disposed in a related-art cuff, and the air bag is formed so as not overlap with the ultrasonic transmissive portion.

According to an aspect of the presently disclosed subject matter, the ultrasonic transmissive portion through which an ultrasonic wave for observing a tissue in a desired region of the living body is transmissible is provided in the body of the cuff. Therefore, an ultrasonic echo image can be produced, and the state of the tissue under pressure can be observed.

The cuff of the presently disclosed subject matter is wrapped around a desired region of the living body, the ultrasonic transmissive portion is caused to face against an observation region, the body of the cuff is fixed to the desired region of the living body by the fixing portion, and the desired region of the living body is pressurized by the pressurizing portion. Then, the ultrasonic wave irradiated from the ultrasonic probe is caused to transmit through the ultrasonic transmissive portion to impinge on the observation region, thereby enabling a measurement of a blood pressure such as the alternative central venous pressure to be accurately performed.

The cuff of the presently disclosed subject matter has a simple configuration in which the ultrasonic transmissive portion is provided in a related-art cuff. When the cuff of the presently disclosed subject matter is used in, for example, breast cancer screening, therefore, the breast cancer screening can be easily performed by the cuff without introducing a dedicated apparatus.

According to an aspect of the presently disclosed subject matter, the object region can be pressurized with constant force. When it is used in, for example, the diagnosis of an ectopic ureterocele, therefore, the flank can be pressed with constant force by the pressurizing portion. Therefore, a clear image of an ureterocele can be smoothly obtained as compared to the case where the pressurization is performed by the hand.

## Claims

1. A cuff comprising:
a belt-like body which is adapted to be wrapped around a desired region of a living body;
a fixing portion which is provided in the belt-like body, and which is configured to fix the belt-like body in a state where it is wrapped around the region of the living body;
a pressurizing portion which is provided in the belt-like body, and to which a fluid pressure is to be supplied to be inflated to pressurize the region of the living body; and
an ultrasonic transmissive portion which is provided in the belt-like body, and through which an ultrasonic wave for observing a tissue in the region of the living body is transmissible,
**characterised in that**
the ultrasonic transmissive portion is configured by providing, in the belt-like body, a window portion formed by a member having ultrasonic transmissive properties.

2. The cuff according to claim 1, wherein the region of the living body is one of four limbs.

3. The cuff according to claim 1, wherein the region of the living body is breast, and the fixing portion is configured to fix the belt-like body in a state where it is wrapped around a chest so as to cover the breast.

4. The cuff according to claim 1, wherein the region of the living body is abdomen.

5. The cuff according to any of claims 1 to 4, wherein the ultrasonic transmissive portion is visually transparent.

6. A tissue observing method comprising:
wrapping the belt-like body of the cuff according to any of claims 1 to 5 around the region of the living body;
fixing, by the fixing portion, the belt-like body in a state where the ultrasonic transmissive portion faces against an observation region of the region of the living body;
supplying the fluid pressure to the pressurizing portion to pressurize the region of the living body; and
irradiating an ultrasonic wave from an ultrasonic probe through the ultrasonic transmissive portion to observe a tissue of the observation region of the region of the living body.

## Patentansprüche

1. Manschette, die Folgendes umfasst:
einen gürtelähnlichen Körper, der dazu angepasst ist, um einen gewünschten Bereich eines lebenden Körpers gewickelt zu werden;
einen Befestigungsabschnitt, der in dem gürtelähnlichen Körper vorgesehen ist und der dazu ausgelegt ist, den gürtelähnlichen Körper in einem Zustand zu befestigen, in welchem er um den Bereich des lebenden Körpers gewickelt ist;
einen Druckbeaufschlagungsabschnitt, der in dem gürtelähnlichen Körper vorgesehen ist und an welchen ein Flüssigkeitsdruck geleitet werden soll, so dass der Druckbeaufschlagungsabschnitt aufgeblasen wird, um den Bereich des lebenden Körpers mit Druck zu beaufschlagen; und
einen ultraschalldurchlässigen Abschnitt, der in dem gürtelähnlichen Körper vorgesehen ist und durch welchen eine Ultraschallwelle zur Beobachtung eines Gewebes im Bereich des lebenden Körpers übertragbar ist,
**dadurch gekennzeichnet, dass** der ultraschalldurchlässige Abschnitt durch die Bereitstellung, in dem gürtelähnlichen Abschnitt, eines Fensterabschnitts ausgelegt ist, der von einem Element mit ultraschalldurchlässigen Eigenschaften gebildet ist.

2. Manschette nach Anspruch 1, wobei der Bereich des lebenden Körpers eine von vier Gliedmaßen ist.

3. Manschette nach Anspruch 1, wobei der Bereich des lebenden Körpers die Brust ist und der Befestigungsabschnitt dazu ausgelegt ist, den gürtelähnlichen Körper in einem Zustand zu befestigen, in welchem er um einen Brustkorb gewickelt ist, so dass die Brust bedeckt ist.

4. Manschette nach Anspruch 1, wobei der Bereich des lebenden Körpers das Abdomen ist.

5. Manschette nach einem der Ansprüche 1 bis 4, wobei der ultraschalldurchlässige Abschnitt optisch transparent ist.

6. Gewebebeobachtungsverfahren, das Folgendes umfasst:
Wickeln des gürtelähnlichen Körpers der Manschette gemäß einem der Ansprüche 1 bis 5 um den Bereich des lebenden Körpers;
Befestigen, durch den Befestigungsabschnitt, des gürtelähnlichen Körpers in einem Zustand, in welchem der ultraschalldurchlässige Abschnitt einem Beobachtungsbereich des Bereichs des lebenden Körpers zugewandt ist;
Leiten des Flüssigkeitsdrucks an den Druckbeaufschlagungsabschnitt, um den Bereich des lebenden Körpers mit Druck zu beaufschlagen; und
Ausstrahlen einer Ultraschallwelle aus einer Ultraschallsonde durch den ultraschalldurchlässigen Abschnitt, um ein Gewebe des Beobachtungsbereichs des Bereichs des lebenden Körpers zu beobachten.

## Revendications

1. Brassard comprenant :
un corps de type ceinture qui est adapté pour être enroulé autour d'une région souhaitée d'un corps vivant ;
une portion de fixation qui est ménagée dans le corps de type ceinture et qui est configurée pour fixer le corps de type ceinture dans un état où il est enroulé autour de la région du corps vivant ;
une portion de mise sous pression qui est ménagée dans le corps de type ceinture et à laquelle une pression de fluide doit être fournie pour qu'elle soit gonflée afin de mettre sous pression la région du corps vivant ; et
une partie de transmission ultrasonore qui est ménagée dans le corps de type ceinture, et à travers laquelle une onde ultrasonore destinée à observer un tissu dans la région du corps vivant peut être transmise,
**caractérisé en ce que**
la portion de transmission ultrasonore est configurée en fournissant, dans le corps de type ceinture, une portion de fenêtre formée par un organe ayant des propriétés de transmission ultrasonore.

2. Brassard selon la revendication 1, dans lequel la région du corps vivant est l'un des quatre membres.

3. Brassard selon la revendication 1, dans lequel la région du corps vivant est la poitrine, et la portion de fixation est configurée pour fixer le corps de type ceinture dans un état où il est enroulé autour d'un buste de façon à couvrir la poitrine.

4. Brassard selon la revendication 1, dans lequel la région du corps vivant est l'abdomen.

5. Brassard selon l'une quelconque des revendications 1 à 4, dans lequel la portion de transmission ultrasonore est visuellement transparente.

6. Procédé d'observation de tissu comprenant :
l'enroulement du corps de type ceinture du brassard selon l'une quelconque des revendications 1 à 5 autour de la région du corps vivant ;
la fixation, par la portion de fixation, du corps de type ceinture dans un état où la portion de transmission ultrasonore est contre une région d'observation de la région du corps vivant ;
la fourniture de la pression de fluide à la portion de mise sous pression pour mettre sous pression la région du corps vivant ; et
l'irradiation d'une onde ultrasonore à partir d'une sonde ultrasonore à travers la portion de transmission ultrasonore pour observer un tissu de la région d'observation de la région du corps vivant.
